# EUROPÄISCHE PATENTSCHRIFT

(11) **EP 3 170 517 B1**
(45) Veröffentlichungstag und Bekanntmachung des Hinweises auf die Patenterteilung: **01.04.2020**
(21) Anmeldenummer: 16197475.3
(22) Anmeldetag: 07.11.2016
(51) Int. Cl.: A61M 1/16, A61M 1/34

(54) **SENSORVORRICHTUNG UND EINE SENSORVORRICHTUNG BEINHALTENDES SYSTEM**
SENSOR DEVICE AND SYSTEM COMPRISING A SENSOR DEVICE
DISPOSITIF DE DÉTECTION ET SYSTÈME COMPRENANT UN DISPOSITIF DE DÉTECTION

(30) Priorität: 23.11.2015 DE 102015120215
(43) Veröffentlichungstag der Anmeldung: 24.05.2017
(73) Patentinhaber: B. Braun Avitum AG, 34212 Melsungen (DE)
(72) Erfinder: ALIC, Miran, 8001 Zürich (CH); STROHHÖFER, Christof, Dr., 34123 Kassel (DE); JANIK, Waldemar, 34212 Melsungen (DE)
(74) Vertreter: Winter, Brandl, Fürniss, Hübner, Röss, Kaiser, Polte - Partnerschaft mbB

(56) Entgegenhaltungen:
- WO-A1-2004/057323
- DE-A1- 1 566 662
- US-A- 3 697 185
- US-A- 3 802 562
- US-A- 4 211 597
- US-A- 6 156 002
- US-A1- 2006 200 064

## Beschreibung

Die Erfindung betrifft eine Sensorvorrichtung und ein die Sensorvorrichtung beinhaltendes System und bezieht sich insbesondere auf eine zentrale Vorrichtung für externe Einzel- und Mehrfachsensoranwendungen zur Messung zumindest einer Messgröße, sowie ein System, das ein zu vermessendes Mittel liefert und die Vorrichtung beinhaltet.

Auf dem Gebiet beispielsweise der Dialyse werden vorwiegend Sensoren zur Überwachung verschiedenster Messparameter eingesetzt und wird beispielsweise mit Hilfe von Extinktionsmessungen in verbrauchter Dialysierflüssigkeit (DF), einem chemischen Abbild des gereinigten Blutes, der Reinigungsgrad des Bluts bestimmt. Die Extinktionsmessungen erfolgen dabei mittels und/oder unter Verwendung eines optischen UV-Transmissionssensors. Der Sensor ist bei bekannten Anordnungen ein fester Bestandteil der Dialysemaschine, so dass jede Dialysemaschine, an der eine gewünschte Größe oder ein entsprechender Messwert auszugeben ist, entwicklungs- und kostenintensiv mit einem oder mehreren solchen Sensor(en) ausgestattet werden muss.

US 3 802 562 A offenbart eine Vorrichtung zum Verteilen einer Dialysierlösung in einer Vorrichtung und ist dazu bestimmt, eine Gruppe von Dialysatoren zu versorgen, während gleichzeitig die Änderungen der Transparenz der verbrauchten Dialysierlösung extern überwacht werden.

WO 2004/057323 A1 bezieht sich auf ein Verfahren und eine Vorrichtung zur Überprüfung der Reaktionsfähigkeit eines elektronischen Sensors für gasförmige und/oder flüssige Medien.

Ein Dialysezentrum kann mehrere solcher Dialysemaschinen gleichzeitig oder zeitlich versetzt einsetzen. Häufig wird dabei Verwurf verbrauchter Dialysierflüssigkeit über eine gemeinsame Leitung abgeführt. Dennoch sind in den mehreren Dialysemaschinen die entsprechenden Sensoren jeweils mehrfach vorzuhalten.

Der Erfindung liegt daher als eine Aufgabe zugrunde, eine Vorrichtung und ein System bereitzustellen, die bzw. das in der Lage ist, in verbrauchter Dialysierflüssigkeit zu messen, ohne eine Vielzahl entsprechender und/oder gleichartiger Sensoren in mehreren Dialysemaschinen vorzuhalten.

Diese Aufgabe wird erfindungsgemäß gelöst durch eine Vorrichtung zur Messung zumindest einer Messgröße mit den Merkmalen des Anspruchs 1, und durch ein System mit zentralisierter Vorrichtung für externe Einzel- und Mehrfach-Sensoreinrichtung mit den Merkmalen des Anspruchs 11.

Der Erfindung liegt die allgemeine Idee zugrunde, in verbrauchter Dialysierflüssigkeit messende Sensoren so aus der Dialysemaschine auszulagern, dass eine zentralisierte Sensorvorrichtung für mehrere Dialysemaschinen gleichzeitig oder zeitlich angepasst genutzt werden kann. Dazu wird erfindungsgemäß die Verwendung der Sensorik so umorganisiert, dass verbrauchte Dialysierflüssigkeit durch eine externe Vorrichtung geleitet wird, welche nur jeweils ein Exemplar eines Sensors beinhaltet. Damit wird die Anzahl eingesetzter Sensoren drastisch reduziert. Um mehrere Dialysemaschinen bedienen zu können, kann die Vorrichtung zusätzlich ein Modul beinhalten, welches durch geeignete Umleitung bzw. Umschaltung von Strömungspfaden von Flüssen, d.h. Strömen von von einer Dialysemaschine ausgegebenen Mitteln (vorwiegend Fluide bzw. Flüssigkeiten wie bspw. verbrauchte Dialysierflüssigkeit einer gewünschten Dialysemaschine, jedoch ohne Beschränkung hierauf) durch den bzw. die Sensoren in der externen Vorrichtung umleiten kann. Das ist insbesondere möglich, weil bekannte Sensoren in einer Dialysemaschine ohnehin nur alle 3 bis 5 Minuten Werte erfassen. In der Zwischenzeit können damit Werte für andere Maschinen aufgezeichnet werden, ohne dass beispielsweise eine Auflösungsverringerung hinzunehmen wäre.

Anstelle einer Konzentration auf eine Herstellung zahlreicher Sensoren zum Verbau in einer Dialysemaschine ist es dadurch vorteilhaft möglich, mit einer externen zentralisierten Sensorvorrichtung die Anzahl systemweit benötigter Sensoren zu reduzieren und Kosten einzusparen. Darüber hinaus wird es möglich, weitere Sensoren für die Messung quantitativer Größen wie beispielsweise Kreatinin und Harnsäure einzusetzen.

Ferner benötigen manche Sensoren eine zusätzliche Vorrichtung für die Zugabe entsprechender Reagenzien. Derartige zusätzliche Vorrichtungen nehmen bei bekannten Anordnungen Raum in einer Dialysemaschine ein und müssen darüber hinaus auch hohe regulatorische Anforderungen erfüllen. Erfindungsgemäß ist es dagegen möglich, alle im Dialysierflüssigkeitsabfluss befindlichen Sensoren in der zentralisierten Vorrichtung unterzubringen. In diesem Fall unterliegen sie als in-vitro Diagnostikum deutlich geringeren regulatorischen Anforderungen. Die erfindungsgemäße Vorrichtung schafft insoweit Raum für Elemente und Komponenten, die tatsächlich individuell in einer Dialysemaschine vorhanden sein müssen. Weitere Vorteile ergeben sich dahingehend, dass auch Low-Cost-Maschinen und/oder Fremdmaschinen, die üblicherweise nicht über eigene und/oder kompatible Sensorik im Abfluss der Dialysemaschine verfügen, ebenfalls an die Vorrichtung anschließbar und im Systemverbund betreibbar sind. Durch bestehende Infrastruktur, wie beispielsweise Datenmanagementsysteme, können außerdem gemessene Werte an eine Dialysemaschine und/oder entsprechende Datenbanken weitergegeben oder übertragen werden.

Im Einzelnen werden die vorstehenden Vorteile realisiert und die Aufgabe gelöst durch eine Vorrichtung zur Messung zumindest einer Messgröße, gemäß Anspruch 1.

Bevorzugt ist der zumindest eine Sensor dazu angeordnet, die zumindest eine Messgröße bezüglich des Mittelstroms entlang einer Fluidstrecke oder einer Luftstrecke zu erfassen.

Bevorzugt bildet die Sensorvorrichtung eine für eine Vielzahl von Dialysemaschinen zentralisierte Sensorvorrichtung, die über eine Flüssigkeitsstrecke oder eine Luftstrecke mit zumindest einem Ausgang und/oder Auslass einer Dialysemaschine verbunden ist. Bevorzugt ist der zumindest eine Sensor dazu angeordnet, eine Clearance und/oder eine Konzentration eines Analyten in dem Mittelstrom als die zumindest eine Messgröße zu erfassen.

Der Sensorvorrichtung ist stromauf eine eine Flusssteuerung bereitstellende Fluidikeinrichtung vorgeordnet, die zumindest einen Mittelstrom durchleitend mit der Sensorvorrichtung verbunden ist, wobei die Fluidikeinrichtung dazu angeordnet ist, selektiv wählbar zumindest einen von einer Vielzahl von Mittelströmen, die von der Vielzahl von Mittelströmen ausgebenden Dialysemaschinen ausgegeben werden, der zumindest einen Sensorvorrichtung zuzuleiten und/oder die Sensorvorrichtung dazu angeordnet ist, selektiv wählbar zumindest eine von einer Vielzahl von Messgrößen einer gewählten Dialysemaschine, deren Mittelstrom der Sensorvorrichtung zugeleitet wird, zu erfassen.

Die Fluidikeinrichtung ist dazu angeordnet, Mittelströme von der Vielzahl von Dialysemaschinen zeitlich gesteuert und in vorbestimmtem Wechsel der Sensorvorrichtung zuzuleiten und/oder einen aktuell gewählten Mittelstrom von einer aktuell gewählten Dialysemaschine für eine vorbestimmte Zeitspanne der Sensorvorrichtung zuzuleiten.

Die vorbestimmte Zeitspanne ist an Erfassungszeitpunkte einer Sensorik in der aktuell gewählten Dialysemaschine anpassbar oder angepasst und sind eine Vielzahl von vorbestimmten Zeitspannen an Erfassungszeitpunkte einer Sensorik jeder der Vielzahl von verbundenen oder verbindbaren Dialysemaschinen anpassbar oder angepasst vorgesehen, die nach Art eines Zeitmultiplex im Wechsel bereitgestellt werden. Bevorzugt ist unter einer Vielzahl von vorbestimmten Zeitspannen zumindest eine vorbestimmte Zeitspanne fester oder einstellbarer Dauer für eine sensoriklose Dialysemaschine vorgesehen.

Bevorzugt ist zu beliebigen Zeitpunkten ein Auswählen einer Dialysemaschine durch einen Anwender erzwingbar und nur durch diesen wieder aufhebbar.

Bevorzugt ist zumindest ein Mehrwegeventil vorgesehen und dazu angeordnet, eingangsseitig mit einer Vielzahl von Mittelströmen ausgebenden Dialysemaschinen verbindbar zu sein, ausgangsseitig mit der Sensorvorrichtung verbunden zu sein, und jeweils einen Mittelstrom der Vielzahl von Mittelströmen ausgebenden Dialysemaschinen der Sensorvorrichtung zuzuleiten. Anstatt des zumindest einen Mehrwegeventils kann auch eine geschickte Kombination einfacher Absperrventile vorgesehen sein, mit welchen die erfindungsgemäße Vorrichtung umgesetzt wird.

Bevorzugt ist eine Schnittstelle zu einer Datenverwaltungs- und/oder Kommunikationseinrichtung für eine Übertragung und/oder Weiterverarbeitung gemessener Daten und/oder Messgrößen in eine zugeordnete Dialysemaschine und/oder Datenbank vorgesehen.

Bevorzugt ist an der Sensorvorrichtung eine Zugabevorrichtung vorgesehen und dazu angeordnet, eine von dem Sensor benötigte Reagenz zuzugeben.

Ein System mit zentralisierter Vorrichtung für externe Einzel- und Mehrfach-Sensoreinrichtung beinhaltet eine zentralisiert angeordnete Vorrichtung zur Messung zumindest einer Messgröße nach einem der Ansprüche 1 bis 10; und zumindest eine Dialysemaschine, die an zumindest einem Auslass den Dialysierflüssigkeitsfluss als Mittelstrom ausgibt, wobei dann, wenn mehrere einen Mittelstrom ausgebende Dialysemaschinen mit der Vorrichtung zur Messung zumindest einer Messgröße verbunden sind, eine der Dialysemaschinen selektiv wählbar und für eine vorbestimmte Zeitspanne mit der Vorrichtung verbindbar ist.

Bevorzugt beinhaltet das System eine Datenverwaltungs- und/oder Kommunikationsvorrichtung, die dazu angeordnet ist, sensierte Information, Daten und/oder Messwerte von der Vorrichtung zur Messung zumindest einer Messgröße zu empfangen.

Bevorzugt ist die Datenverwaltungs- und/oder Kommunikationsvorrichtung dazu angeordnet, von der Vorrichtung zur Messung zumindest einer Messgröße empfangene Information, Daten und/oder Messwerte an eine vorbestimmte eine einen Mittelstrom ausgebende Dialysemaschine zu übertragen und/oder in eine Datenbank zu speichern.

Bevorzugt ist die Datenverwaltungs- und/oder Kommunikationsvorrichtung dazu angeordnet, von einer einen Mittelstrom ausgebenden Dialysemaschine ausgegebene Steuerungs- und/oder Regelungsanweisungen an die Vorrichtung zur Messung zumindest einer Messgröße zu übertragen.

Bevorzugt beinhaltet das System eine Anzeigeeinrichtung zur Anzeige von von der Vorrichtung zur Messung zumindest einer Messgröße sensierter Information, Daten und/oder Messwerte und/oder von von einer einen Mittelstrom ausgebenden Dialysemaschine ausgegebener Informationen oder Anweisungen mit Bezug zu systembezogenen Steuerungs- und/oder Regelungsfunktionen.

Die Erfindung wird nachstehend anhand bevorzugter Ausführungsbeispiele unter Bezugnahme auf die beigefügten Zeichnungen näher beschrieben. Es zeigen:
Fig. 1 in schematischer Darstellung ein System zur zeitlich angepassten Vermessung verschiedener Größen in verbrauchter Dialysierflüssigkeit über systematische Verbindungen der Flüssigkeitsausgänge von Dialysemaschinen mit einer zentralisierten Sensorvorrichtung gemäß einem Ausführungsbeispiel;
Fig. 2 eine schematische Darstellung einer Fluidik, welche dazu angeordnet ist, Flüsse aus verschiedenen Dialysemaschinen zu steuern und zu verwalten und die in dem Ausführungsbeispiel nach Fig. 1 verwendbar ist; und
Fig. 3 eine schematische Darstellung einer Modifikation der Fluidik nach Fig. 2, welche dazu angeordnet ist, Flüsse aus verschiedenen Dialysemaschinen zu steuern und zu verwalten und die in dem Ausführungsbeispiel nach Fig. 1 verwendbar ist.

In der nachfolgenden Figurenbeschreibung werden in den einzelnen Figuren gleiche oder gleichwirkende Elemente und Komponenten mit denselben Bezugszeichen bezeichnet und zweckmäßig nicht redundant beschrieben. In Fällen, in welchen ein nachfolgendes Ausführungsbeispiel funktionell zumindest einem vorangehenden entspricht, d.h. entsprechende Funktionen, Anordnungen und/oder Betriebsabläufe gleichermaßen umfasst sind, wird zweckmäßig nur auf Unterschiede eingegangen.

Gemäß den nachfolgenden Ausführungsbeispielen sind allgemein eine Vorrichtung und ein die Vorrichtung beinhaltendes System zur Vermessung verschiedener Größen (Leitfähigkeit, Temperatur, pH-Wert, Kt/V, Konzentrationen von Analyten wie Kreatinin bzw. Creatinine, Harnsäure und dergleichen und von Elektrolyten wie Natrium und dergleichen) mit Hilfe einer zentralisierten Sensorvorrichtung über den Flüssigkeitsausgang oder eine Luftstrecke mit der Dialysemaschine verbunden. Durch geeignete Flusssteuerung können verschiedene Messgrößen ausgewählter Maschinen erfasst werden. Eine Datenmanagement- und/oder Kommunikationsapplikation ermöglicht den Transfer bzw. die Übertragung gemessener Informationen, Daten und/oder Werte in die Dialysemaschine (z.B. zur Anzeige auf einer Anzeigeeinrichtung oder beispielsweise als Steuer- und Regelbefehle) und/oder in eine Datenbank.

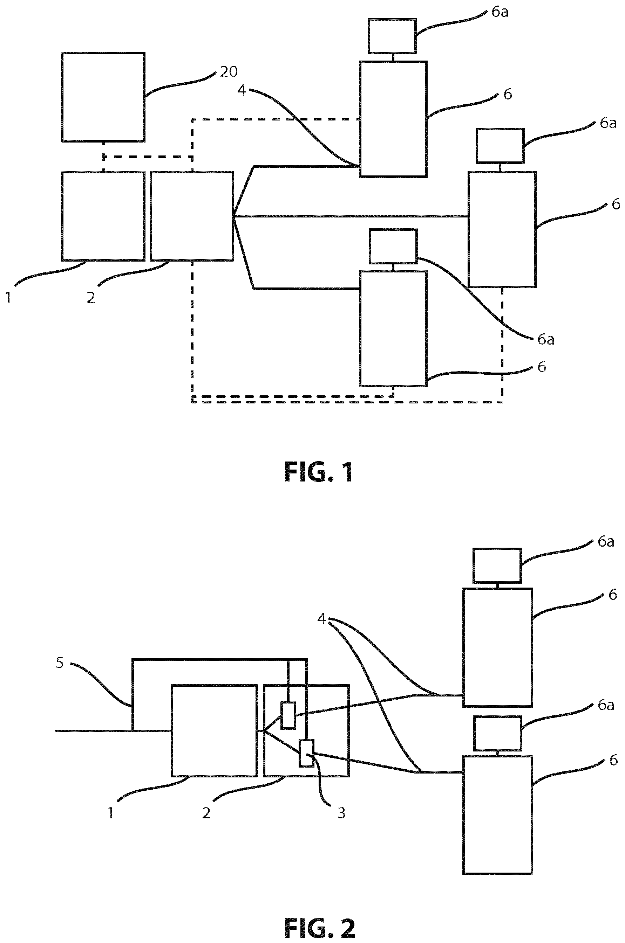

Fig. 1 zeigt in schematischer Darstellung ein System zur zeitlich angepassten Vermessung verschiedener Größen in verbrauchter Dialysierflüssigkeit über systematische Verbindungen der Flüssigkeitsausgänge von Dialysemaschinen mit einer zentralisierten Sensorvorrichtung (Stand-Alone-Sensor). Eine zusätzliche Anbindung an ein Netzwerk für eine Datenkommunikation zwischen einem Sensor, einem Server, Datenbanken und zumindest einer Dialysemaschine ist in durchbrochener Linie ebenfalls angedeutet. Durch die Sensorvorrichtung gesammelte Werte können auf diese Weise zur Weiterverarbeitung und an beispielsweise einen Bildschirm der Dialysemaschine zur Anzeige weitergegeben werden.

Im Einzelnen weisen in Fig. 1 drei Dialysemaschinen 6 mit jeweils einer Eingabe- und/oder Anzeigeeinrichtung 6a, beispielsweise einem Bildschirm, einer Tastatur und dergleichen, jeweils einen Auslass 4 für Dialysierflüssigkeit auf. In Fig. 1 ist aus Gründen der Übersichtlichkeit der Darstellung nur ein solcher Auslass mit dem Bezugszeichen 4 bezeichnet. Außerdem besteht keine Beschränkung auf die Anzahl von 3 Dialysemaschinen. Auslegungsabhängig sind nahezu beliebig viele Dialysemaschinen in das System gemäß dem Ausführungsbeispiel integrierbar. Die einzelnen Auslässe 4 jeder der Dialysemaschinen 6 werden separat jeweils Einlässen einer Fluidikeinrichtung 2, die eine Einrichtung für intelligentes Fluidmanagement bildet, zugeführt. Die Fluidikeinrichtung 2 weist intern zumindest ein noch zu beschreibendes Mehrwegeventil auf, das dazu angeordnet ist, Fluidstrecken bzw. Fluidleitungen gesteuert derart zu öffnen und zu schließen, dass die Fluidikeinrichtung 2 jeweils zumindest einen selektierten, d.h. ausgewählten, Fluid- oder Flüssigkeitsstrom, der einen Strom eines von einer Dialysemaschine ausgegebenen Mittels bildet, durchleitet und nicht selektierte, d.h. nicht ausgewählte, Fluidströme von anderen Dialysemaschinen sperrt. Der durchgeleitete Fluidstrom wird an einem Auslass der Fluidikeinrichtung 2 ausgegeben und einer Sensorvorrichtung 1 zur dortigen Vermessung zugeführt.

Fig.1 zeigt somit ein System aus beispielsweise drei Dialysemaschinen 6 (wobei jedoch nahezu beliebig viele Dialysemaschinen anbindbar sind und insoweit keine Beschränkung bezüglich der Anzahl von Dialysemaschinen besteht), welche jeweils über ihren (Dialysier-) Flüssigkeitsausgang 4 mit der Sensorvorrichtung 1 (dem Stand-Alone-Sensor) verbunden sind. Die Auswahl einer der zu vermessenden Dialysemaschinen 6 kann durch geeignete Auslegung einer der Sensorvorrichtung 1 stromauf vorgelagerten bzw. vorgeschalteten Flusssteuerung, d.h. Fluidikeinrichtung 2, mit intelligentem Fluidmanagement erfolgen. Es wird angemerkt, dass die Fluidikeinrichtung 2 optional ausgebildet sein kann, d.h. eine Zuschaltung und/oder Wegschaltung von Fluidströmen beispielsweise vermittels geeigneter Ventileinrichtungen und dergleichen auch direkt an der Dialysemaschine 6 erfolgen kann oder die Fluidikeinrichtung 2 in die Sensorvorrichtung 1 integriert ausgebildet sein kann.

Die Sensorvorrichtung 1 kann ferner einen oder mehrere verschiedene (nicht dargestellte) Sensoren sowie eine dazu notwendige Mechanik und Elektronik, wie beispielsweise an sich bekannte Pumpen, Ansteuerungen, Recheneinheiten und dergleichen, aufnehmen. Mittels einer Kommunikations- und/oder Datenmanagement- bzw. Datenverwaltungseinrichtung 20 oder einer entsprechenden Anwendung können weiters Informationen an die Dialysiermaschine gesendet werden. Derartige Informationen können beispielsweise ein erfasster Konzentrationswert oder ein Clearance- bzw. Kt/V-Wert sein. Es ist aber auch möglich, Steuerbefehle mitzusenden bzw. zu übertragen, um beispielsweise einen Blutfluss zu steuern und es dadurch anderen Verfahren zu ermöglichen, etwa auch blutseitige Konzentrationen zu bestimmen.

Erfasste Werte stehen der Dialysemaschine 6 zur Verfügung und können entweder zur Anzeige auf dem Bildschirm 6a ausgegeben werden und/ oder aber in eine (nicht dargestellte) Datenbank geschrieben werden. Die so erhaltenen Daten erlauben in diesem Fall auch eine Auswertung über einen längeren Zeitraum.

Fig. 2 zeigt eine schematische Darstellung einer Fluidik, welche dazu angeordnet ist, Flüsse aus verschiedenen Dialysemaschinen 6 zu steuern und zu verwalten und die in dem Ausführungsbeispiel nach Fig. 1 verwendbar ist.

Im Einzelnen sind in Fig. 2 zwei Dialysemaschinen 6 mit jeweils zugehöriger Bedien- und/oder Anzeigeeinrichtung 6a mit jeweils einem Dialysierflüssigkeitsauslass 4 dargestellt. Jeder von den Dialysemaschinen 6 an deren Dialysierflüssigkeitsauslässen 4 als Mittelstrom ausgegebene Dialysierflüssigkeitsfluss wird einem entsprechenden Einlass der Fluidikeinrichtung 2 zugeführt. In der Fluidikeinrichtung 2 ist zumindest ein Multi- bzw. Mehrwegeventil 3 vorgesehen, das dazu angeordnet ist, in vorbestimmter Weise gesteuert und für jeweils feste oder anpassbare Zeitspannen jeden einzelnen Dialysierflüssigkeitsfluss separat durchzuleiten oder zu sperren.

Beispielsweise kann gemäß Fig. 2 selektiv die von der oberen Dialysemaschine 6 ausgegebene Dialysierflüssigkeit zu der Sensorvorrichtung 1 durchgeleitet werden, während die von der unteren Dialysemaschine 6 ausgegebene Dialysierflüssigkeit an dem Mehrwegeventil 3 gesperrt und somit nicht zu der Sensorvorrichtung 1 durchgeleitet wird. Nicht zu der Sensorvorrichtung 1 durchgeleitete Dialysierflüssigkeitsströme können dann über beispielsweise an dem Mehrwegeventil 3 ableitende Verwurfleitungen 5 aus der Fluidikeinrichtung 2 ausgeleitet werden, um eine fortleitende Ausleitung von Dialysierflüssigkeit auch aus einer nicht ausgewählten Dialysemaschine 6 zu gewährleisten.

Vorzugsweise werden dabei Zeitspannen in Übereinstimmung mit einem Zeitverlauf von Werteerfassungen an einer jeweiligen Dialysemaschine 6 gemäß einem dort erfolgenden Therapieverlauf festgelegt oder gewählt. In anderen Worten erfolgt eine Durchleitung von Dialysierflüssigkeit einer bestimmten Dialysemaschine 6 zur Vermessung in der Sensorvorrichtung 1 vorzugsweise während Zeitspannen oder Zeitpunkten, während welchen an der bestimmten Dialysemaschine 6 eine Aufnahme bzw. Erfassung von Werten erfolgen soll (beispielsweise etwa alle 3 bis 5 Minuten). Zu anderen Zeiten kann Dialysierflüssigkeit einer anderen Dialysemaschine 6 vermessen werden. Beispielsweise können dazu mehrere Dialysemaschinen 6 so betrieben werden bzw. therapieren, dass sie jeweils erforderliche Werte mit geeignetem Zeitversatz aufnehmen.

Es versteht sich hierbei, dass Komponenten und/oder Systemteile auch mehrfach ausgelegt oder vorgesehen sein können. Falls beispielsweise im Rahmen bestehender Zykluszeiten eine Anzahl bereits verbundener Dialysemaschinen 6 den Anschluss weiterer Dialysemaschinen 6 nicht mehr erlaubt, kann/können eine zweite Fluidikeinrichtung 2 und/oder eine zweite Sensorvorrichtung 1 dazu angeordnet werden, Mittelströme aus den weiteren Dialysemaschinen 6 zu vermessen.

Gemäß dem in Fig. 2 vereinfacht gezeigten Ausführungsbeispiel einer Flusssteuerung bzw. Fluidik sind somit mehrere Dialysemaschinen 6 mit einer alleinstehenden Sensorvorrichtung 1 verbindbar. Zwar wurde in Fig. 2 aus Gründen der Übersichtlichkeit auf eine Darstellung digitaler Verbindungen verzichtet, jedoch versteht sich, dass Informationen zwischen einer entsprechenden Dialysemaschine 6 und der alleinstehenden Sensorvorrichtung 1 transferiert werden können.

Den Kern des vorstehend beschriebenen intelligenten Fluidmanagements in Übereinstimmung mit der Fluidikeinrichtung 2 bildet zumindest ein Multiwegeventil 3, das gemäß dem Ausführungsbeispiel in einer Variante mit zwei Wegen dargestellt ist. Der Ausgang bzw. Auslass 4 für Dialysierflüssigkeit einer Dialysemaschine 6 ist über die Fluidikeinrichtung 2 mit dem zumindest einen Mehrwegeventil 3 verbunden. Damit besteht die Möglichkeit, die Flüsse in der Maschine zu steuern. Über eine (nicht gezeigte) digitale Schnittstelle kann nun entschieden werden, für welche Maschine die Dialysierflüssigkeit untersucht werden soll. Die restlichen Wege werden in den Ausfluss verworfen. Die gemessenen Werte können dann zu der entsprechenden Maschine zur Darstellung und/oder Weiterverarbeitung übermittelt werden.

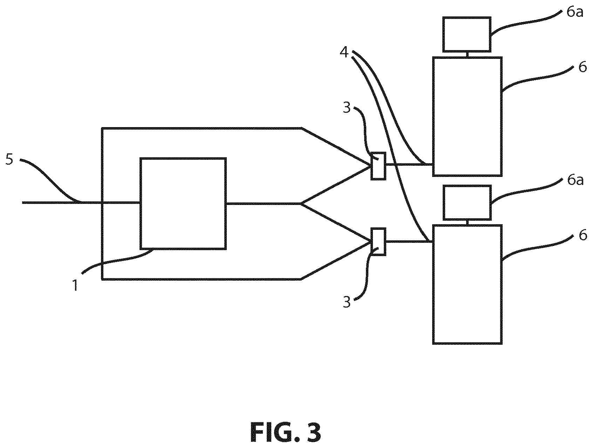

Fig. 3 zeigt eine schematische Darstellung einer Modifikation der Fluidik nach Fig. 2, welche dazu angeordnet ist, Flüsse aus verschiedenen Dialysemaschinen zu steuern und zu verwalten und die in dem Ausführungsbeispiel nach Fig. 1 verwendbar ist. Gemäß Fig. 3 ist das zumindest eine Mehrwegeventil 3 direkt an dem Auslass 4 der Dialysiermaschine 6 angeordnet. Weiter vorteilhaft ist auf diese Weise unter Beibehaltung des vorstehend beschriebenen Messprinzips eine Strangführung flexibler gestaltbar.

Erfindungsgemäß vorteilhaft werden Einsparungen von Kosten für den Bau vieler Sensoren erzielt, ist der Einsatz mehrerer Sensoren zur Erhebung bzw. Erfassung verschiedenster Größen wie Kt/V, Kreatinin, Harnsäure, aber auch für enzymatische Tests, möglich, sind geringere regulatorische Anforderungen durch eine mögliche Klassifizierung als In-Vitro-Diagnostikum zu erfüllen und profitieren auch Dialysemaschinen, bei welchen keine eigene Sensorik vorgesehen ist, von den genannten Vorteilen der beschriebenen Sensorik.

Vorstehend wurden somit eine Vorrichtung zur Messung zumindest einer Messgröße und ein System, das die Vorrichtung für externe Einzel- und Mehrfach-Sensoreinrichtung zentralisiert beinhaltet, beschrieben. Die Vorrichtung beinhaltet eine Sensorvorrichtung mit zumindest einem Sensor, die über zumindest eine mittelführende Erfassungsstrecke mit zumindest einer von einer Vielzahl von zumindest einen Dialysierflüssigkeitsfluss als Mittelstrom ausgebenden Dialysemaschinen verbindbar ist, wobei der zumindest eine Sensor dazu angeordnet ist, selektiv zumindest eine Messgröße bezüglich zumindest eines von einer der Vielzahl von Dialysemaschinen ausgegebenen Mittelstroms zu erfassen. Das System umfasst darüber hinaus zumindest eine Dialysemaschine, die an zumindest einem Auslass den Dialysierflüssigkeitsfluss als Mittelstrom ausgibt, wobei dann, wenn mehrere einen Mittelstrom ausgebende Dialysemaschinen mit der Vorrichtung zur Messung zumindest einer Messgröße verbunden sind, eine der Dialysemaschinen selektiv wählbar und für eine vorbestimmte Zeitspanne mit der Vorrichtung verbindbar ist.

Es versteht sich, dass die Erfindung nicht auf die beschriebenen Ausführungsbeispiele, insbesondere nicht auf die lediglich beispielhaft herangezogene Dialysemaschine und die beispielhaft angegebene Dialysierflüssigkeit und die in deren Kontext angegebenen Zahlenwerte und Größenordnungen beschränkt ist, sondern dass sich innerhalb des durch die nachstehenden Ansprüche definierten Schutzumfangs für den Fachmann gleichwohl naheliegend Modifikationen und Äquivalente ergeben können.

## Patentansprüche

1. Vorrichtung zur Messung zumindest einer Messgröße, beinhaltend:
eine Sensorvorrichtung (1) mit zumindest einem Sensor, die über zumindest eine mittelführende Erfassungsstrecke mit einer von einer Vielzahl von jeweils einen Dialysierflüssigkeitsfluss als Mittelstrom ausgebenden Dialysemaschinen (6) verbindbar ist, wobei der zumindest eine Sensor dazu angeordnet ist, selektiv zumindest eine Messgröße bezüglich des von der einen Dialysemaschine (6) ausgegebenen Mittelstroms zu erfassen; und
eine Fluidikeinrichtung (2), die der Sensorvorrichtung (1) stromauf vorgeordnet, über eine Vielzahl von Mittelströmen führenden Strecken mit der Vielzahl von Dialysemaschinen (6) verbindbar und genau einen Mittelstrom zu der Sensorvorrichtung (1) durchleitend mit der Sensorvorrichtung (1) verbunden ist, wobei
die Fluidikeinrichtung (2) dazu angeordnet ist, durch selektives Wählen eines der Vielzahl von Mittelströmen, die von der Vielzahl von Dialysemaschinen (6) über die Vielzahl von Mittelströme führenden Strecken ausgegeben werden, und Zuleiten des gewählten einen Mittelstroms zu dem zumindest einen Sensor der Sensorvorrichtung (1) eine Flusssteuerung für die Dialysierflüssigkeitsflüsse bereitzustellen, und
die Fluidikeinrichtung (2) dazu angeordnet ist, Mittelströme von der Vielzahl von Dialysemaschinen (6) zeitlich gesteuert und in vorbestimmtem Wechsel der Sensorvorrichtung (1) zuzuleiten, und/oder einen aktuell gewählten Mittelstrom von einer aktuell gewählten Dialysemaschine (6) für eine vorbestimmte Zeitspanne der Sensorvorrichtung (1) zuzuleiten, **dadurch gekennzeichnet, dass**
die vorbestimmte Zeitspanne an Erfassungszeitpunkte einer Sensorik in der aktuell gewählten Dialysemaschine (6) anpassbar oder angepasst ist und eine Vielzahl von vorbestimmten Zeitspannen an Erfassungszeitpunkte einer Sensorik jeder der Vielzahl von verbundenen oder verbindbaren Dialysemaschinen (6) anpassbar oder angepasst vorgesehen sind und nach Art eines Zeitmultiplex im Wechsel bereitgestellt werden.

2. Vorrichtung nach Anspruch 1, bei der der zumindest eine Sensor dazu angeordnet ist, die zumindest eine Messgröße bezüglich des Mittelstroms entlang einer Fluidstrecke oder einer Luftstrecke zu erfassen.

3. Vorrichtung nach Anspruch 1 oder 2, bei der die Sensorvorrichtung (1) eine für eine Vielzahl von Dialysemaschinen (6) zentralisierte Sensorvorrichtung (1) bildet, die über eine Flüssigkeitsstrecke mit zumindest einem Ausgang (4) und/oder Auslass der Dialysemaschine (6) verbunden ist.

4. Vorrichtung nach einem der Ansprüche 1 bis 3, bei der der zumindest eine Sensor dazu angeordnet ist, eine Clearance und/oder eine Konzentration eines Analyten in dem Mittelstrom als die zumindest eine Messgröße zu erfassen.

5. Vorrichtung nach einem der vorangehenden Ansprüche, bei der die Sensorvorrichtung (1) dazu angeordnet ist, selektiv wählbar zumindest eine von einer Vielzahl von Messgrößen einer gewählten Dialysemaschine (6), deren Mittelstrom der Sensorvorrichtung (1) zugeleitet wird, zu erfassen.

6. Vorrichtung nach einem der Ansprüche 1 bis 5, bei der unter einer Vielzahl von vorbestimmten Zeitspannen zumindest eine vorbestimmte Zeitspanne fester oder einstellbarer Dauer für eine sensoriklose Dialysemaschine (6) vorgesehen ist.

7. Vorrichtung nach einem der Ansprüche 1 bis 6, bei der zu beliebigen Zeitpunkten ein Auswählen einer Dialysemaschine (6) durch einen Anwender erzwingbar ist und nur durch diesen wieder aufhebbar ist.

8. Vorrichtung nach einem der vorangehenden Ansprüche, bei der zumindest ein Mehrwegeventil (3) oder eine Kombination einfacher Absperrventile vorgesehen und dazu angeordnet ist, eingangsseitig mit einer Vielzahl von Mittelströmen ausgebenden Dialysemaschinen (6) verbindbar zu sein, ausgangsseitig mit der Sensorvorrichtung (1) verbunden zu sein und jeweils einen Mittelstrom der Vielzahl von Mittelströmen ausgebenden Dialysemaschinen (6) der Sensorvorrichtung (1) zuzuleiten.

9. Vorrichtung nach einem der vorangehenden Ansprüche, bei der eine Schnittstelle zu einer Datenverwaltungs- und/oder Kommunikationseinrichtung (20) für eine Übertragung und/oder Weiterverarbeitung gemessener Daten und/oder Messgrößen in eine zugeordnete Dialysemaschine (6) und/oder Datenbank vorgesehen ist.

10. Vorrichtung nach einem der vorangehenden Ansprüche, bei der an der Sensorvorrichtung (1) eine Zugabevorrichtung vorgesehen und dazu angeordnet ist, eine von dem Sensor benötigte Reagenz zuzugeben.

11. System mit zentralisierter Vorrichtung für externe Einzel- und Mehrfach-Sensoreinrichtung, beinhaltend:
eine zentralisiert angeordnete Vorrichtung zur Messung zumindest einer Messgröße nach einem der vorangehenden Ansprüche; und
zumindest eine Dialysemaschine (6), die an zumindest einem Auslass (4) den Dialysierflüssigkeitsfluss als Mittelstrom ausgibt, wobei
dann, wenn mehrere einen Mittelstrom ausgebende Dialysemaschinen (6) mit der Vorrichtung zur Messung zumindest einer Messgröße verbunden sind, eine der Dialysemaschinen (6) selektiv wählbar und für eine vorbestimmte Zeitspanne mit der Vorrichtung verbindbar ist.

12. System nach Anspruch 11, ferner beinhaltend:
eine Datenverwaltungs- und/oder Kommunikationsvorrichtung (20), die dazu angeordnet ist, sensierte Informationen, Daten und/oder Messwerte von der Vorrichtung zur Messung zumindest einer Messgröße zu empfangen.

13. System nach Anspruch 12, bei dem die Datenverwaltungs- und/oder Kommunikationsvorrichtung (20) dazu angeordnet ist, von der Vorrichtung zur Messung zumindest einer Messgröße empfangene Informationen, Daten und/oder Messwerte an eine vorbestimmte einen Mittelstrom ausgebende Dialysemaschine (6) zu übertragen und/oder in eine Datenbank zu speichern.

14. System nach Anspruch12 oder 13, bei dem die Datenverwaltungs- und/oder Kommunikationsvorrichtung (20) dazu angeordnet ist, von einer einen Mittelstrom ausgebenden Dialysemaschine (6) ausgegebene Steuerungs- und/oder Regelungsanweisungen an die Vorrichtung zur Messung zumindest einer Messgröße zu übertragen.

15. System nach einem der Ansprüche11 bis 14, ferner beinhaltend:
eine Anzeigeeinrichtung (6a) zur Anzeige von von der Vorrichtung zur Messung zumindest einer Messgröße sensierter Informationen, Daten und/oder Messwerte und/oder von von einer einen Mittelstrom ausgebenden Dialysemaschine (6) ausgegebener Information oder Anweisungen mit Bezug zu systembezogenen Steuerungs- und/oder Regelungsfunktionen.

## Claims

1. Device for measuring at least one measurand, comprising:
a sensor device (1) which comprises at least one sensor and, via at least one medium-carrying detection path, can be connected to one of a plurality of dialysis machines (6) which each output a dialysis liquid flow as a medium flow, the at least one sensor being arranged to selectively detect at least one measurand with respect to the medium flow output by said one dialysis machine (6); and
a fluidic unit (2) which is arranged upstream of the sensor device (1), can be connected to the plurality of dialysis machines (6) via a plurality of paths carrying medium flows and is connected to the sensor device (1) so as to pass on exactly one medium flow to the sensor device (1), wherein
the fluidic unit (2) is arranged to provide a flow control for dialysis liquid flows, namely by selectively choosing one of the plurality of medium flows output by the plurality of dialysis machines (6) via the plurality of paths carrying medium flows and by supplying the one selected medium flow to the at least one sensor of the sensor device (1), and
the fluidic unit (2) is arranged to supply the sensor device (1) with medium flows from the plurality of dialysis machine (6) in a timely controlled manner and in a predetermined switching scheme, and/or to supply the sensor device (1) with a currently selected medium flow from a currently selected dialysis machine (6) for a predetermined period of time, **characterized in that**
the predetermined period of time can be adapted or is adapted to detection times of a sensor system in the currently selected dialysis machine (6), and a plurality of predetermined periods of time are provided to be adaptable or to be adapted to detection times of a sensor system of each of the plurality of connected or connectable dialysis machines (6) and are made available alternately in the manner of a time multiplex.

2. Device according to claim 1, in which the at least one sensor is arranged to detect the at least one measurand with respect to the medium flow along a fluid path or an air path.

3. Device according to claim 1 or 2, in which the sensor device (1) constitutes a sensor device (1) which is centralized for a plurality of dialysis machines (6) and is connected to at least one output (4) and/or outlet of the dialysis machine (6) via a liquid path.

4. Device according to one of claims 1 to 3, in which the at least one sensor is arranged to detect a clearance and/or a concentration of an analyte in the medium flow as the at least one measurand.

5. Device according to one of the preceding claims, in which the sensor device (1) is arranged to detect, in a selectively selectable manner, at least one of a plurality of measurands of a selected dialysis machine (6) whose medium flow is fed to the sensor device (1).

6. Device according to one of claims 1 to 5, in which among a plurality of predetermined periods of time at least one predetermined period of time having a fixed or adjustable duration is provided for a sensor-less dialysis machine (6).

7. Device according to one of claims 1 to 6, in which a selection of a dialysis machine (6) can be enforced at any points in time by a user and can only be canceled by the latter.

8. Device according to one of the preceding claims, in which at least one multi-port valve (3) or a combination of simple shut-off valves is provided and is arranged to be connectable at the input side with a plurality of dialysis machines (6) outputting medium flows, to be connected to the sensor device (1) at the output side, and to supply the sensor device (1) with one medium flow each from the plurality of dialysis machines (6) outputting medium flows.

9. Device according to one of the preceding claims, in which an interface to a data management and/or communication device (20) is provided for a transmission and/or further processing of measured data and/or measurands in an associated dialysis machine (6) and/or a database.

10. Device according to one of the preceding claims, in which an addition device is provided on the sensor device (1) and arranged to add a reagent required by the sensor.

11. System comprising a centralized device for an external single or multiple sensor device, comprising:
a centrally arranged device for measuring at least one measurand according to one of the preceding claims; and
at least one dialysis machine (6) outputting the dialysis fluid flow as medium flow at at least one outlet (4), wherein
one of the dialysis machines (6) can be selectively selected and can be connected to the device for a predetermined period of time if several dialysis machines (6) outputting a medium flow are connected to the device for measuring at least one measurand.

12. System according to claim 11, further comprising:
a data management and/or communication device (20) arranged to receive captured information, data and/or measured values from the device for measuring at least one measurand.

13. System according to claim 12, in which the data management and/or communication device (20) is arranged to transfer information, data and/or measured values received from the device for measuring at least one measurand to a predetermined dialysis machine (6) outputting a medium flow and/or to store these in a database.

14. System according to claim 12 or 13, in which the data management and/or communication device (20) is arranged to transfer control and/or closed-loop control instructions, which are output by a dialysis machine (6) outputting a medium flow, to the device for measuring at least one measurand.

15. System according to one of claims 11 to 14, further comprising:
a display device (6a) for displaying information, data and/or measured values captured by the device for measuring at least one measurand and/or for displaying information or instructions, relevant to system-related control and/or closed-loop control functions, output by a dialysis machine (6) outputting a medium flow.

## Revendications

1. Dispositif de mesure d'au moins une grandeur de mesure, incluant :
un dispositif capteur (1) avec au moins un capteur, qui peut être relié par l'intermédiaire d'au moins une section de détection guidant du fluide à une machine de dialyse parmi une pluralité de machines de dialyse (6) fournissant respectivement un flux de liquide de dialyse comme courant de fluide, dans lequel l'au moins un capteur est agencé pour détecter sélectivement au moins une grandeur de mesure concernant le courant de fluide fourni par la machine de dialyse (6) considérée ; et
un appareil fluidique (2), qui est placé en amont du dispositif capteur (1), qui peut être relié par l'intermédiaire d'une pluralité de sections guidant des courants de fluide à la pluralité de machines de dialyse (6) et qui est relié au dispositif capteur (1) en conduisant exactement un courant de fluide au dispositif capteur (1), dans lequel
l'appareil fluidique (2) est agencé pour mettre à disposition une commande de flux pour les flux de liquide de dialyse en choisissant sélectivement un courant de fluide parmi la pluralité de courants de fluide, qui sont fournis par la pluralité de machines de dialyses (6) par l'intermédiaire d'une pluralité de sections guidant des courants de fluide, et en conduisant l'un courant de fluide choisi à l'au moins un capteur du dispositif capteur (1), et
l'appareil fluidique (2) est agencé pour conduire des courants de fluide de la pluralité de machines de dialyse (6), selon une commande temporelle et une alternance prédéterminée, au dispositif capteur (1), et/ou pour conduire un courant de fluide actuellement choisi d'une machine de dialyse (6) actuellement choisie, pour un laps de temps prédéterminé, au dispositif capteur (1), **caractérisé en ce que**
le laps de temps prédéterminé peut être ou est adapté à des instants de détection d'un système capteur dans la machine de dialyse (6) actuellement choisie et une pluralité de laps de temps prédéterminés sont prévus de manière à pouvoir être ou à être adaptés à des instants de détection d'un système capteur de chaque machine de dialyse parmi la pluralité de machines de dialyse (6) reliées ou pouvant être reliées et sont mis à disposition en alternance à la manière d'un multiplexage temporel.

2. Dispositif selon la revendication 1, dans lequel l'au moins un capteur est agencé pour détecter l'au moins une grandeur de mesure concernant le courant de fluide le long d'une section de fluide ou d'une section d'air.

3. Dispositif selon la revendication 1 ou 2, dans lequel le dispositif capteur (1) forme un dispositif capteur (1) qui est centralisé pour une pluralité de machines de dialyse (6) et qui est relié par l'intermédiaire d'une section de liquide à au moins une sortie (4) et/ou évacuation de la machine de dialyse (6).

4. Dispositif selon l'une quelconque des revendications 1 à 3, dans lequel l'au moins un capteur est agencé pour détecter en tant que l'au moins une grandeur de mesure une clairance et/ou une concentration d'un analyte dans le courant de fluide.

5. Dispositif selon l'une quelconque des revendications précédentes, dans lequel le dispositif capteur (1) est agencé pour détecter, d'une manière pouvant être choisie sélectivement, au moins une grandeur de mesure parmi une pluralité de grandeurs de mesure d'une machine de dialyse (6) sélectionnée dont le courant de fluide est conduit au dispositif capteur (1).

6. Dispositif selon l'une quelconque des revendications 1 à 5, dans lequel, parmi une pluralité de laps de temps prédéterminés, au moins un laps de temps prédéterminé d'une durée fixe ou réglable est prévu pour une machine de dialyse (6) sans système capteur.

7. Dispositif selon l'une quelconque des revendications 1 à 6, dans lequel, à des instants quelconques, une sélection d'une machine de dialyse (6) peut être contrainte par un utilisateur et ne peut être à nouveau annulée que par celui-ci.

8. Dispositif selon l'une quelconque des revendications précédentes, dans lequel au moins une soupape à plusieurs voies (3) ou une combinaison de soupapes d'arrêt simples est prévue et est agencée pour pouvoir être reliée côté entrée à une pluralité de machines de dialyse (6) fournissant des courants de fluide, pour être reliée côté sortie au dispositif capteur (1) et pour conduire respectivement un courant de fluide de la pluralité de machines de dialyse (6) fournissant des courants de fluide au dispositif capteur (1).

9. Dispositif selon l'une quelconque des revendications précédentes, dans lequel une interface vers un dispositif de gestion de données et/ou de communication (20) en vue d'une transmission et/ou d'un traitement ultérieur de données mesurées et/ou de grandeurs de mesure dans une machine de dialyse (6) associée et/ou dans une base de données associée est prévue.

10. Dispositif selon l'une quelconque des revendications précédentes, dans lequel, au niveau du dispositif capteur (1), un dispositif d'ajout est prévu et agencé pour ajouter un réactif nécessaire au capteur.

11. Système avec dispositif centralisé pour appareil capteur externe unique et multiple, incluant :
un dispositif agencé de manière centralisée et destiné à la mesure d'au moins une grandeur de mesure selon l'une quelconque des revendications précédentes ; et
au moins une machine de dialyse (6), qui fournit au niveau d'au moins une sortie (4) le flux de liquide de dialyse comme courant de fluide, dans lequel
si plusieurs machines de dialyse (6) fournissant un courant de fluide sont reliées au dispositif destiné à la mesure d'au moins une grandeur de mesure, l'une des machines de dialyse (6) peut être reliée, d'une manière pouvant être choisie sélectivement et pour un laps de temps prédéterminé, au dispositif.

12. Système selon la revendication 11, incluant en outre :
un dispositif de gestion de données et/ou de communication (20) qui est agencé pour recevoir du dispositif des informations, données et/ou valeurs de mesure captées en vue de la mesure d'au moins une grandeur de mesure.

13. Système selon la revendication 12, dans lequel le dispositif de gestion de données et/ou de communication (20) est agencé pour transmettre des informations, données et/ou valeurs de mesure reçues du dispositif en vue de la mesure d'au moins une grandeur de mesure à une machine de dialyse (6) prédéterminée fournissant un courant de fluide et/ou pour les mémoriser dans une base de données.

14. Système selon la revendication 12 ou 13, dans lequel le dispositif de gestion de données et/ou de communication (20) est agencé pour transmettre au dispositif des instructions de commande et/ou de régulation fournies par une machine de dialyse (6) fournissant un courant de fluide en vue de la mesure d'au moins une grandeur de mesure.

15. Système selon l'une quelconque des revendications 11 à 14, incluant en outre :
un appareil d'affichage (6a) en vue de l'affichage d'informations, données et/ou valeurs de mesure captées par le dispositif en vue de la mesure d'au moins une grandeur de mesure et/ou d'informations ou instructions qui sont fournies par une machine de dialyse (6) fournissant un courant de fluide et qui concernent des fonctions de commande et/ou de régulation se rapportant au système.
